# EUROPEAN PATENT APPLICATION

(11) **EP 4 450 159 A1**
(43) Date of publication of application: **23.10.2024**
(21) Application number: 23305608.4
(22) Date of filing: 19.04.2023
(51) Int. Cl.: B01J 23/75, B01J 23/755, B01J 35/00, B01J 37/18, B01J 37/34, B01J 19/08, B01J 23/745, C07C 209/16, C07C 211/07

(54) **LOW TEMPERATURE AMINATION OF ALIPHATIC ALCOHOLS USING MAGNETIC NANOPARTICLES UNDER MAGNETIC INDUCTION**

(71) Applicant: SPECIALTY OPERATIONS FRANCE, 69003 Lyon (FR); LE CENTRE NATIONAL DE LA RECHERCHE SCIENTIFIQUE (CNRS), 75794 Paris, Cedex 16 (FR); Institut National des Sciences Appliquees de Toulouse (INSA Toulouse), 31077 Toulouse Cedex 4 (FR); UNIVERSITE TOULOUSE III - PAUL SABATIER, 31062 Toulouse Cedex 9 (FR)
(72) Inventor: VARELA-IZQUIERDO, Victor, 31077 Toulouse (FR); MUSTIELERES, Irene, 31077 Toulouse (FR); CHAUDRET, Bruno, 31077 Toulouse (FR)
(74) Representative: Valentino, Cédric

(57) **Abstract**

The present invention relates to a process for low temperature amination of aliphatic alcohols by using catalytic magnetic nanoparticles under magnetic induction. Furthermore, the present invention relates to novel magnetic nanoparticles suitable for said amination process. The nanoparticles have a copper shell and a nickel-copper alloy or a nickel-cobalt alloy as core material.

## Description

### TECHNICAL FIELD

The present invention relates to a process for low temperature amination of aliphatic alcohols by using catalytic magnetic nanoparticles under magnetic induction. Furthermore, the present invention relates to novel magnetic nanoparticles suitable for said amination process.

### TECHNICAL BACKGROUND

Alkylated amines are building block for many chemical compounds such as fungicides, pharmaceuticals, detergents, agrochemicals, dyes, pesticides and herbicides. Thus, synthesis of alkylated amines, especially tertiary amines, is a paramount goal for the chemical industry.

Alkylated amines are generally prepared from alkyl halides or alkyl sulfates which are toxic and produce large environmental wastes. For this reason, the development of green synthesis of tertiary amines is a fundamental approach in this topic. Therefore, there has been a growing interest in the development of sustainable synthetic strategies, among which the catalytic amination of primary alcohols for preparing long-chain aliphatic amines is a non-toxic alternative since water is the only stoichiometric by-product with an exceptional atom-economy yield. Unfortunately, the alcohol is not reactive enough, therefore a metal catalyst is commonly employed to dehydrate it and generate a more reactive carbonyl group in a borrowing hydrogen (BH) process, which generally is the rate determining step. Regarding homogeneous catalysis, BH reactions has been well developed using mainly Ru catalysts and other transition metals such as Ir. Conversely, non-noble metals such as copper and nickel are generally used as catalysts in heterogeneous catalysis for this process, although ruthenium catalysts are also employed. Another system for alcohol amination to prepare alkylated amines as described in the prior art is for example a self-suppling system for active hydrogen with a CuNi colloidal catalyst. In this process, the hydrogen produced in dehydrogenation of dodecyl alcohol is subsequently consumed in the hydrogenation of the enamine, contrary to other systems for which the presence of hydrogen is required. Nevertheless, there is still the need for developing improved catalytic amination of primary alcohols for preparing long-chain aliphatic amines.

In recent years, for heterogeneous catalytic reactions in gas phase such as Fischer-Tropsch reaction, CO₂ hydrogenation, methane reforming or alkane dehydrogenation promising results regarding magnetically induced catalytic reactions have been developed. In this respect, ferromagnetic nanoparticles (NPs) can produce heat when exposed to an alternating magnetic field working at high frequency. Moreover, magnetic heating has also been used in liquid phase reactions such as C-C coupling, amide condensation, and more recently in hydrogenation and hydrodeoxygenation reactions. In particular, Cu-decorated iron carbide nanoparticles as catalysts for the selective hydrodeoxygenation of aromatic aldehydes using magnetic induction heating are described in the prior art. Hence, copper nanoparticles seem to be a feasible choice for the two catalytic steps in alcohol amination reaction since the Cu sites is suitable for performing the alcohol oxidation to aldehyde and for enamine hydrogenation step.

However, in particular the Cu-decorated iron carbide nanoparticles are difficult to prepare. Furthermore, in general, Cu NPs are limited by their poor thermal stability and their deactivation. However, usually the catalytic amination of aliphatic alcohols requires high temperature (>150 °C) (see e.g. Baiker, J. Kijenski, Cat. Reviews: Science and Engineering, 1985, 27, pages 653-657).

A further problem of the catalytic amination of aliphatic alcohols is the formation of undesired side products at the high temperatures, i.e. low selectivity of the catalytic amination of alcohols.

Therefore, an aim of the present invention is to provide a catalytic system which allows an amination of aliphatic alcohols in a sufficient amount and with a high selectivity.

### SUMMARY OF THE INVENTION

It has now been discovered that alkyl amines can be prepared in high yield with low formation of by-products by reacting an aliphatic alcohol with an alkyl amine in the presence of catalytic magnetic nanoparticles heated by magnetic induction.

The catalytic magnetic nanoparticles have preferably a magnetic core, which is preferably a bimetallic compound of for example cobalt and nickel or of iron and nickel, and a catalytic metal shell, preferably a shell of copper.

Furthermore, the invention relates to magnetic nanoparticles comprising a bimetallic core of CoₓNi_{y}, wherein x is from 1 to 9, preferably equal to 4, and y = 10-x, and a shell of copper; or comprising a bimetallic core of FeₓNi_{y}, wherein x is from 1 to 9, preferably equal to 3, and y = 10 - x, and a shell of a catalytic metal, preferably of copper.

### BRIEF DESCRIPION OF THE DRAWINGS

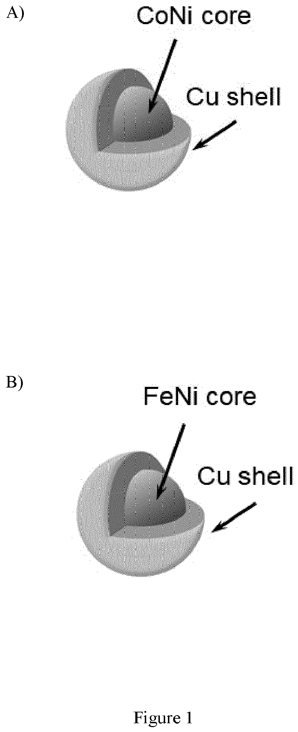

**Figure 1**: schematic drawing of the magnetic nanoparticles (NPs) according to the invention showing A) a core of Co₄Ni₆ with a copper shell and B) a core of Fe₃Ni₇ with a copper shell;
**Figure 2**: TEM images for Co₄Ni₆@Cu₁₀ NPs (Mean size = 18.3 ± 3.7 nm);
**Figure 3**: TEM images for Fe₃Ni₇@Cu₁₀ NPs (Mean size = 24.8 ± 4.6 nm);
Figure 4: A) Powder XRD (X-Ray Diffraction) spectrum of Co₄Ni₆@Cu₁₀ NPs. B) Powder XRD (X-Ray Diffraction) spectrum of Fe₃Ni₇@Cu₁₀ NPs;
**Figure 5**: SAR (Specific Absorption Rate) of Co₄Ni₆@Cu₁₀ NPs determined by calorimetry: 10 mg of NPs dispersed in 0.5 mL of mesitylene (Cp (specific heat capacity) = 400 J.kg⁻¹.K⁻¹ for Co₄Ni₆@Cu₁₀ alloy, Cp = 1750 J.kg⁻¹.K⁻¹ for mesitylene, Cp = 4186 J.kg⁻¹.K⁻¹ for water and Cp = 720 J.kg-1.K-1 for glass); and
**Figure 6**: SAR of Fe₃Ni₇@Cu₁₀ NPs determined by calorimetry: 10 mg of NPs dispersed in 0.5 mL of mesitylene (Cp = 415 J.kg⁻¹.K⁻¹ for Fe₃Ni₇@Cu₁₀ alloy, Cp = 1750 J.kg⁻¹.K⁻¹ for mesitylene, Cp = 4186 J.kg⁻¹.K⁻¹ for water and Cp = 720 J.kg⁻¹.K⁻¹ for glass).

### DETAILED DESCRIPTION OF THE INVENTION

Before the process and the catalytic system of the invention will be described in detail, it is to be understood that this invention is not limited to specific process conditions or specific embodiments of the catalytic system described herein, since such conditions and embodiments may, of course, vary.

It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise. By way of example, "a compound" means one compound or more than one compound.

The terms "containing", "contains" and "contained of" as used herein are synonymous with "including", "includes" or " comprising", "comprises", and are inclusive or open-ended and do not exclude additional, non-recited members, elements or process steps. It will be appreciated that the terms "containing", "contains", "comprising", "comprises" and "comprised of" as used herein comprise the terms "consisting of", "consists" and "consists of".

Throughout this application, the term "about" is used to indicate that a value includes the standard deviation of error for the device or method being employed to determine the value.

As used herein, the term "average" refers to number average unless indicated otherwise.

As used herein, the terms "% by weight", "wt.- %", "weight percentage", or "percentage by weight" are used interchangeably. The same applies to the terms "% by volume", "vol.- %", "vol. percentage", or "percentage by volume", or "% by mol", "mol- %", "mol percentage", or "percentage by mol".

The recitation of numerical ranges by endpoints includes all integer numbers and, where appropriate, fractions subsumed within that range (e.g. 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g. from 1.0 to 5.0 includes both 1.0 and 5.0). Any numerical range recited herein is intended to include all sub-ranges subsumed therein.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different alternatives, embodiments and variants of the invention are defined in more detail. Each alternative and embodiment so defined may be combined with any other alternative and embodiment, and this for each variant unless clearly indicated to the contrary or clearly incompatible when the value range of a same parameter is disjoined. In particular, any feature indicated as being preferred or advantageous may be combined with any other feature or features indicated as being preferred or advantageous.

Furthermore, the particular features, structures or characteristics described in present description may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and from different embodiments, as would be understood by those in the art.

The present invention relates to a process for producing alkyl amines by reacting an aliphatic alcohol with an alkyl amine in the presence of catalytic magnetic nanoparticles heated by magnetic induction.

As used herein, the term "catalytic" means having a catalytic activity for the reaction of the invention i.e. for the reaction of an aliphatic alcohol with an alkyl amine.

According to the invention, the aliphatic alcohols can be linear or branched, but preferably the aliphatic alcohols are linear aliphatic alcohols. The aliphatic alcohols have preferably 7 to 12, preferably 8 to 10, carbon atoms. Additionally, it is preferred that the aliphatic alcohols are primary alcohols. In a preferred embodiment, the aliphatic alcohol is a primary aliphatic alcohol containing 7 to 12 carbon atoms, more preferably 8 to 10 carbon atoms, like heptanol, octanol or dodecanol.

The alkyl amines according to the invention are preferably secondary alkyl amines, more preferably dialkyl amines. Furthermore, even though the alkyl groups of the dialkyl amine can be different, according to the invention, it is preferred that secondary alkyl amines are symmetric, i.e., the two alkyl moieties of the secondary aliphatic alkyl amine are identical. The alkyl group(s) of the alkyl amine preferably comprises one to six, more preferably two to four carbon atoms. In a preferred embodiment, the alkyl amine is a dialkyl amine, preferably with alkyl groups comprising one to six carbon atoms, preferably two to four carbon atoms. Examples of aliphatic alkyl amines suitable for the process of the invention are dimethylamine, dipropylamine and dibutylamine.

In a preferred embodiment of the invention, the aliphatic alcohol is chosen from heptanol, octanol and dodecanol, and the alkyl amine is chosen from dimethylamine, dipropylamine and dibutylamine.

The nanoparticles according to the invention contain at least one metal or alloy that has a catalytic activity for the reaction of the invention i.e. for the reaction of an aliphatic alcohol with an alkyl amine. Such metals/alloys are known from those skilled in the art for instance through the article to Baiker, J. Kijenski mentioned above. For instance copper, nickel, cobalt, irridium and ruthenium are often used as catalytic metals. Especially copper gives good results in the frame of the invention.

The catalytic magnetic particles of the invention can be made of a magnetic alloy also having a catalytic activity for the reaction of the invention. An example of such an alloy is a Ni/Co alloy like Co₄Ni₆.

The catalytic magnetic nanoparticles according to the invention have preferably a magnetic core, which heats the reaction medium when an alternating magnetic field working at high frequency is applied on the catalytic magnetic nanoparticles, and a catalytic metal shell, which favours the catalytic activity.

Preferably, the magnetic core is a bimetallic compound, more preferably a bimetallic compound of iron and nickel or of cobalt and nickel. The shell according to the invention is preferably a copper shell. Hence, catalytic magnetic nanoparticles suitable for carrying out the process according to the invention are for example nanoparticles having a bimetallic core of FeₓNi_{y} or CoₓNi_{y} and a shell of copper, indicated as FeₓNi_{y}@Cu or CoₓNi_{y}@Cu, wherein x and y are as defined above.

In a preferred embodiment of the invention, the bimetallic core of the catalytic magnetic nanoparticles are made of Co₄Ni₆@Cu₁₀ or Fe₃Ni₇@Cu₁₀.

The bimetallic compound, which forms the core of the magnetic nanoparticles according to this embodiment of the invention, can be produced by any suitable method known in the art, for example as described in:
- Angew. Chem. Int. Ed.2022, 61, e202207301 (Organometallic Synthesis of Magnetic Metal Nanoparticles)
- Angew. Chem. Int. Ed.2020, 59, 6187 -6191 (Engineering Iron-Nickel Nanoparticles for Magnetically Induced CO2Methanation in Continuous Flow).

In particular, it is preferred that the metals, like Ni, Co and/or Fe, forming the bimetallic compound, react in form of metal complexes having in addition to the metal atom ligands selected from the group consisting of amidinato-, η² pyrazolato-, amines (HDA), carbenes (NHC), phosphines (dppb) and guanidine ligands. Suitable complexes for the production of bimetallic core compounds according to the invention are for example {Ni[ⁱPrNC(CH₃)NⁱPr]₂}, {Co[N(SiMe₃)₂]₂(thf)},{Fe[N(SiMe₃)₂]₂Ni(cod)2], [Co(cod)(cot)], {Co[N(SiMe3)2]2}2, {Co[iPrNC(CH3)NiPr]2}, {Co[iPrNC(nBu)NiPr]2}, {Fe2[iPrNC(CH3)NiPr]4}, {Fe[nBu(CH3) nBu]2} [Fe2(mesityl)4], and [Fe(C9H7)2]. The appropriate metal complexes are dissolved in a solvent, for example toluene, mesitylene or xylenes in the presence of an organic acid, preferably a fatty acid, like palmitic acid. The reaction may be carried out under hydrogen pressure, for example at 2 to 4 bar, preferably at 3 bar, at a temperature of 110 to 130 °C for 20 to 30 hours, under stirring, for instance magnetic stirring. Afterwards, the bimetallic compound is preferably separated, for instance by magnetic decantation. It is then preferably washed, for instance with a solvent like toluene, and then preferably dried, for instance under vacuum.

Afterwards, the catalytic metal layer, preferably a copper layer, is applied on the produced bimetallic compound. In this reaction step, the metal, for example copper, is also preferably used in form of complex, for example {Cu₂[ⁱPrNC(CH₃)NⁱPr]₄}, [Cu(mesityl)2] and{Cu2[iPrNC(nBu)NiPr]4}. Preferably, similar conditions as those which are used for producing the bimetallic compound, are used for the applying the catalytic metal layer on the bimetallic compound. Subsequently, the magnetic nanoparticles according to the invention are preferably separated, washed and dried as described above for the bimetallic compound.

The magnetic nanoparticles according to the invention have preferably a mean particle size of 1 to 100 nm, preferably of 5 to 50 nm, more preferably of 10 to 35 nm, determined by TEM imaging (Transmission Electron Microscopy, which uses a high-energy electron beam to form high-resolution images of objects on the nanoscale).

The use of the magnetic nanoparticles as described herein generally causes the temperature of the reactor in which the amination reaction is taking place, to rise from room temperature to a temperature generally comprised between 50 and 160°C, even between 90 and 160 °C, and sometimes between 95 °C and 155 °C. The heat is generated by applying an alternating magnetic field on the magnetic nanoparticles. Preferably, the amplitude of the magnetic field is adapted so as to generate the desired reaction temperature, which actually depends on the SAR of the magnetic particles.

Therefore, the reaction mixture of aliphatic alcohol, alkylamine and magnetic nanoparticles is preferably placed in the centre of an induction coil, for example in the centre of a 300 kHZ induction coil. The induction coil may be equipped with a user interface, where the software permits to continuously monitor and adjust the percentage of magnetic field amplitude applied. In case of the apparatus used in the Examples, the relation between the power in kW and the magnetic field amplitudes in % and mT is shown in Table 1 below.

**Table 1**

| **Field (%)** | **Power (kW)** | **Field Amplitude (mT)** |
|---|---|---|
| 100 | 12.9 | 65 |
| 80 | 10.8 | 60 |
| 60 | 8.2 | 53 |
| 40 | 5.5 | 44 |
| 20 | 2.8 | 33 |
| 10 | 1.5 | 24 |
| 5 | 0.8 | 15 |

In the magnetic field, the magnetic nanoparticles reorganize to form chains along the magnetic field direction. This reorganization produces heat, which is sufficient to carried out the amination reaction of the invention.

According to the invention, preferably the aliphatic alcohols and the alkyl amines are used in stoichiometric amounts in the process, although varying amounts of educts can be used to improve yields and/or selectivity of the process.

The magnetic nanoparticles are preferably used in an amount such that the total number of moles of metal relative to the total number of moles of alcohol is between 2 to 20 %, preferably between 3 and 15 %.

The process of the invention is conducted preferably for 1.5 to 20 hours, more preferably for 2 to 15 hours, even more preferably for 5 to 10 hours.

By using the process of the invention, preferably tertiary amines in high yields are produced, like N,N-dibutylheptanamine N,N-dimethyldodecanamine, N,N-dipropyldodecanamine and N,N-dibutyldodecanamine. The produced amines can be dried under vacuum for further use.

Despite a side trans-alkylation reaction of the dialkylamine, the process of the invention has high conversion rate and selectivity compared to the reaction carried out under conventional heating.

The conversion rate of the process of the invention may be 99% or higher. Generally, the conversion rate is from 5 up to 99 %, often of 20 to 90 %, more often from 30 to 85 %.

The selectivity of the process of the invention may be 99 % or higher. Generally, the selectivity is between 75 and 99%, often between 80 and 98%.

The inventors found that by carrying out the amination process of the invention as described above, the use of an additional solvent (like water or THF) or working under hydrogen pressure (e.g. 3 bar) to obtain the high conversion rates and/or selectivity is not necessary. They also found that the use of a drying agent, like CaCO₃, to remove the water produced in the reaction is not necessary (same conversion and selectivity were observed with and without).

The present invention is further illustrated by the following examples. It should be understood that the following examples are for illustration purposes only and are not used to limit the present invention thereto.

### EXAMPLES

### Synthesis of the magnetic nanoparticle cores

**Co₄Ni₆ (1)**: In a glovebox {Co[N(SiMe₃)₂]₂(thf)} (418 mg, 0.96 mmol) and {Ni[ⁱPrNC(CH₃)NⁱPr]} (475 mg, 1.4 mmol) were dissolved separately in 4 and 6 mL of degassed mesitylene respectively. Both solutions were mixed in a 100 mL Fisher Porter (FP) bottle. Then, palmitic acid (593 mg, 2.3 mmol) was dissolved in 8 mL of mesitylene and was added to the mixture under vigorous magnetic stirring. The FP bottle was pressurized with 3 bar of H₂ and stirred at RT (Romm Temperature) for 40 min. Then, the reaction mixture was heated at 120 °C for 24 hours under magnetic stirring. The nanoparticles (NPs) were recovered by magnetic decantation, washed with toluene (3 x 5 mL) and dried under vacuum. Co₄Ni₆ NPs were obtained as black powder (ca. 135 mg)

The NPs metal content determined by ICP (Inductively Coupled Plasma) was the following: Co: 32 wt.%; Ni: 48 wt.%.

**Fe₃Ni₇ (2)**: In a glove box, {Ni[ⁱPrNC(CH3)NⁱPr]} (692 mg, 2.00 mmol) and {Fe[N(SiMe₃)₂]₂}₂ (301 mg, 0.40 mmol) were dissolved separately in 8 and 4 mL of degassed mesitylene respectively. Both solutions were mixed in a 100 mL FP bottle. Then, palmitic acid (185 mg, 0.72 mmol) dissolved in 8 mL of mesitylene was added to the mixture under vigorous magnetic stirring. The FP bottle was pressurized with 3 bar of H₂ and heated at 150 °C for 24 hours under magnetic stirring. The NPs were recovered by magnetic decantation, washed with toluene (3 x 5 mL) and dried under vacuum.

Fe₃Ni₇ NPs were obtained as a black powder (ca. 130 mg). Metal content determined by ICP: Fe: 28 wt.%; Ni: 67 wt.%.

### Synthesise of the magnetic nanoparticles according to the invention

**Co₄Ni₆@Cu₁₀ (3):** In a glovebox, 100 mg of NPs (1) (ca. 1.3 mmol of metal) were dispersed in 10 mL of mesitylene in a 180 mL FP bottle. Then, a solution of {Cu₂[ⁱPrNC(CH₃)NⁱPr]₄} (327.6 mg, 0.80 mmol) in 8 mL of mesitylene was added. Afterwards, palmitic acid (410.2 mg, 1.6 mmol) was dissolved in 16 mL of mesitylene and was added to the mixture under vigorous magnetic stirring. The FP bottle was pressurized with 3 bar of H₂ and the reaction was heated at 80 °C for 3 hours and then, at 120 °C for 24 hours under magnetic stirring. The NPs were recovered by magnetic decantation, washed with toluene (4 x 5 mL) and dried under vacuum. NPs 3 were recovered as a reddish black powder (ca. 150 mg). Metal content determined by ICP: Co 16 wt.%; Ni: 24 wt.%; Cu 49 wt. %.

**Fe₃Ni₇@Cu₁₀ (4):** In a glovebox, 50 mg of NPs (2) (ca. 0.86 mmol of metal) were dispersed in 5 mL of mesitylene in a 100 mL FP bottle. Then, a solution of {Cu₂[ⁱPrNC(CH₃)NiPr]₄} (163.8mg, 0.40 mmol) in 5 mL of mesitylene was added. Afterwards, palmitic acid (205.1 mg, 0.8 mmol) was dissolved in 10 mL of mesitylene and was added to the mixture under vigorous magnetic stirring. The FP bottle was pressurized with 3 bar of H₂ and the reaction was heated at 120 °C for 24 hours under magnetic stirring. The NPs were recovered by magnetic decantation, washed with toluene (3 x 5 mL) and dried under vacuum. NPs 4 were recovered as a black powder (ca. 78 mg). Metal content determined by ICP: Fe: 12 wt.%; Ni: 37 wt.%; Cu: 46 wt.%.

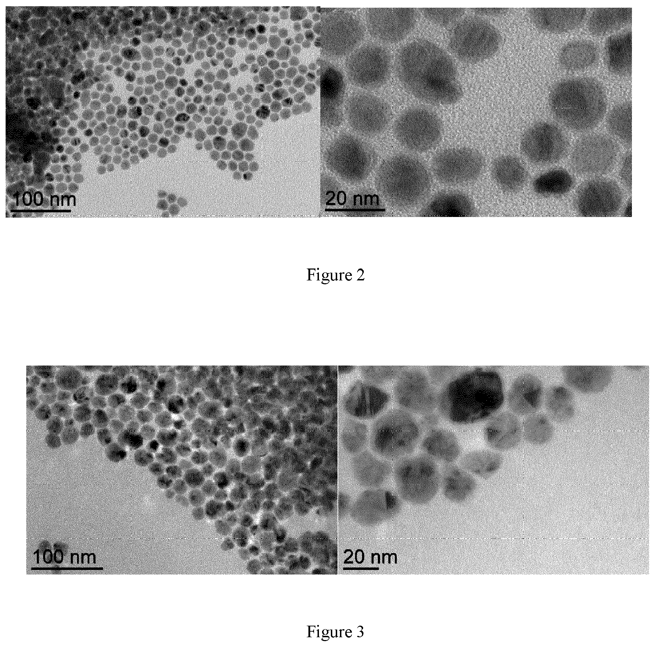

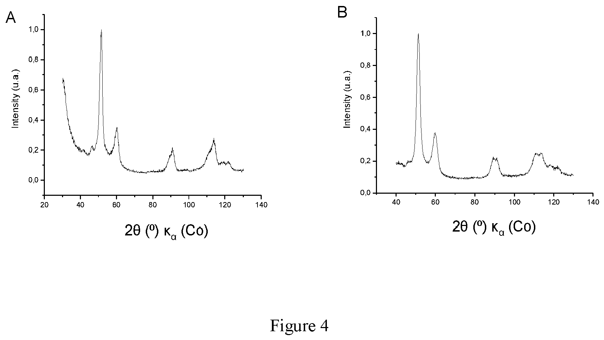

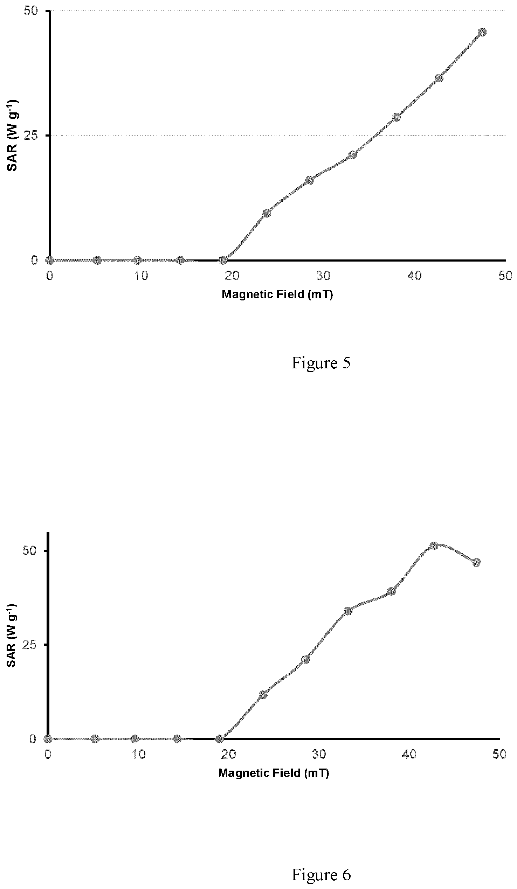

The TEM, XRD and SAR properties of NPs (3) and (4) are presented in Figures 2 to 6.

### EXAMPLE 1 - Amination of 1-dodecanol with nBu₂NH:

On a schlenk line, dodecanol (1.12 mL, 5 mmol) was introduced into a 100 mL Fisher Porter (FP) bottle and three vacuum/argon cycles were performed. Afterwards, n-dibutylamine (0.84 mL, 5 mmol), 15 or 45 mg of catalyst (Co₄Ni₆ (1), Fe₃Ni₇ (2), Co₄Ni₆@Cu₁₀ (3) or Fe₃Ni₇@Cu₁₀ (4)) and dodecane (227 µL, 1 mmol, as internal standard) were added. The FP was closed, removed from the glovebox and immediately placed in the the center of the magnetic coil and the amplitude of magnetic field settled to the desired value for the required time. At the end of the reaction, the FP was cooled down and pressure was released (in some reactions, manometer pressure was > 1 bar. The gas was analysed by GC-MS and identified as H₂ - due to oxidation of the alcohol to aldehyde). Temperature was recorded on the surface of the external glass of the FP with an IR camera after 1 h of reaction (entries 1-8). In the blank reaction (entries 9-11), the FP was placed in an oil bath instead of the coil at the indicated temperature.

For the isolation of the tertiary amines, 8 mL of diethylether were added and the catalyst was separated by magnetic decantation. Then, MgSO₄ was added and stirred for 20 minutes. The crude was filtered with Celite^{®} and the extracted liquid was analysed by GC-MS. Conversion was calculated with the relative area of the remaining alcohol/amine. Selectivity was calculated with the relative area of the product peaks.

The results are shown in Table 2 below. As can be seen in this table, Co₄Ni₆ nanoparticles have a catalytic activity on their own, while Fe₃Ni₇ nanoparticles are not catalytic magnetic particles according to the invention. This might be due to the fact that such nanoparticles have an Iron-rich shell (see Angew. Chem. Int. Ed.2020, 59, 6187 -6191 (Engineering Iron-Nickel Nanoparticles for Magnetically Induced CO2Methanation in Continuous Flow), while Iron is not reported as being a good catalyst for amination reactions (see see e.g. Baiker, J. Kijenski, Cat. Reviews: Science and Engineering, 1985, 27, pages 653-657).

**TABLE 2**

| **Entry** | **Cat.*** | **Cu mol % (M mol %)**** | **Magnetic Field (mT)** | **Time (h)** | **Temp. (°C)** | **Conv. (%)** | **Selec. (%)** |
|---|---|---|---|---|---|---|---|
| 1 | **1** | (4.3) | 39 | 2 | 150 | >99 | 60 |
| 2 | **2** | (4.3) | 39 | 2 | 128 | <1 | - |
| 3 | **3** | 2.3 (4.3) | 39 | 2 | 96 | 21 | 98 |
| 4 | **3** | 2.3 (4.3) | 49 | 2 | 107 | 32 | 97 |
| 5 | **4** | 2.2 (4.7) | 65 | 2 | 115 | 7 | >99 |
| 6 | **3** | 2.3 (4.3) | 49 | 8 | 96 | 72 | 80 |
| 7 | **4** | 2.2 (4.7) | 65 | 8 | 95 | 43 | >99 |
| 8 | **3** | 6.8 (12.8) | 49 | 3 | 151 | >99 | 85 |
| 9 | **3** | 6.8 (12.8) | Conventional heating | 3 | 115 | <1 | - |
| 10 | **3** | 6.8 (12.8) | Conventional heating | 3 | 140 | 45 | - |
| 11 | **3** | 6.8 (12.8) | Conventional heating | 3 | 160 | 30 | 20 |

| | | | | | | | |
|---|---|---|---|---|---|---|---|
| (^{∗}: 1 = Co₄Ni₆, 2 = Fe₃Ni₇, 3 = Co₄Ni₆@Cu₁₀, 4 = Fe₃Ni₇@Cu₁₀. ^{∗∗}: total number of moles of metal relative to alcohol; these terms are also used with respect to all other examples following below) | | | | | | | |

### EXAMPLE 2 - Amination of heptanol with nBu₂NH:

On a schlenk line, heptanol (1.12 mL, 5 mmol) was introduced into a 100 mL Fisher Porter (FP) bottle and three vacuum/argon cycles were performed. Afterwards, n-dibutylamine (0.84 mL, 5 mmol), 45 mg of catalyst (Co₄Ni₆@Cu₁₀ (3)) and dodecane (227 µL, 1 mmol, as internal standard) were added. The FP was closed, removed from the glovebox and immediately placed in the the center of the magnetic coil and the amplitude of magnetic field settled to the desired value for the required time. At the end of the reaction, the FP was cooled down and pressure was released. Temperature was recorded on the surface of the external glass of the FP with an IR camera after 1 h of reaction.

For the isolation of the tertiary amines, 8 mL of diethylether were added and the catalyst was separated by magnetic decantation. Then, MgSO₄ was added and stirred for 20 minutes. The crude was filtered with Celite^{®} and the extracted liquid was analysed by GC-MS. The result is shown in Table 3 below.

**Table 3**

| **Entry** | **Cat.** | **Cu mol % (M mol %)** | **Magnetic Field (mT)** | **Time (h)** | **Temp. (°C)** | **Conv. (%)** | **Selec. (%)** |
|---|---|---|---|---|---|---|---|
| 1 | 3 | 6.8 (12.8) | 49 | 3 | - | >99 | 75 |

### EXAMPLE 3 - Amination of dodecanol with DMA (40 %wt. in water):

On a schlenk line, dodecanol (1.12 mL, 5 mmol) was introduced into a 100 mL Fisher Porter (FP) bottle and three vacuum/argon cycles were performed. Afterwards, DMA (40 %wt. in water, 0.63 mL, 5 mmol), 15, 30 or 45 mg of catalyst (Co₄Ni₆@Cu₁₀ (3)) and dodecane (227 µL, 1 mmol, as internal standard) were added. The FP was closed, removed from the glovebox and immediately placed in the the center of the magnetic coil and the amplitude of magnetic coil settled to the desired value for the required time. At the end of the reaction, the FP was cooled down and pressure was released. Temperature was recorded on the surface of the external glass of the FP with an IR camera after 1 h of reaction.

For the isolation of the tertiary amines, 3 mL of diethylether were added and the organic phase was separated from the aqueous phase three times. In addtion, remaining catalyst was separated by magnetic decantation and MgSO₄ were added and stirred for 20 minutes. The crude was filtered with Celite^{®} and the extracted liquid was analysed by GC-MS. The results are shown in Table 4 below.

| **Entry** | **Cat.** | **Cu mol % (M mol %)** | **Magnetic Field (mT)** | **Time (h)** | **Temp. (°C)** | **Conv. (%)** | **Selec. (%)** |
|---|---|---|---|---|---|---|---|
| 1 | **3** | 2.3 (4.3) | 49 | 2 | 89 | 19 | >99 |
| 2 | **3** | 6.8 (12.8) | 49 | 2 | 101 | 36 | >99 |
| 3 | **3** | 6.8 (12.8) | 49 | 5 | 118 | 91 | 88 |
| 4 | **3** | 6.8 (12.8) | 49 | 8 | 115 | >99 | 87 |
| 5 | **3** | 4.6 (8.6) | 49 | 8 | 108 | 75 | 92 |
| 6 | **3** | 4.6 (8.6) | 39 | 12 | 95 | 92 | 92 |

| | | | | | | | |
|---|---|---|---|---|---|---|---|
| Table 4 | | | | | | | |

### EXAMPLE 4 - Amination of 1-dodecanol and DMA (2 M in THF):

On a schlenk line, dodecanol (1.12 mL, 5 mmol) was introduced into a 100 mL Fisher Porter (FP) bottle and three vacuum/argon cycles were performed. Afterwards, DMA (2 M in THF, 2.5 mL, 5 mmol), 15 or 45 mg of catalyst (Co₄Ni₆@Cu₁₀ (3) or Fe₃Ni₇@Cu₁₀ (4)) and dodecane (227 µL, 1 mmol, as internal standard) were added. The FP was closed, removed from the glovebox and immediately placed in the centre of the magnetic coil and the amplitude of magnetic field settled to the desired value for the required time. At the end of the reaction, the FP was cooled down and pressure was released. Temperature was recorded on the surface of the external glass of the FP with an IR camera after 1 h of reaction.

For the isolation of the tertiary amines, 6.5 mL of diethylether were added and the catalyst was separated by magnetic decantation. Then, MgSO₄ were added and stirred for 20 minutes. The crude was filtered with Celite^{®} with a thin layer of charcoal on the top and the liquid was analysed by GC-MS. Conversion was calculated with the relative area of the remaining alcohol/amine. Selectivity was calculated with the relative area of the product peaks. The results are shown in Table 5 below.

**Table 5**

| **Entry** | **Cat.** | **Cu mol % (M mol %)** | **Magnetic Field (mT)** | **Time (h)** | **Temp. (°C)** | **Conv. (%)** | **Selec. (%)** |
|---|---|---|---|---|---|---|---|
| 1 | **3** | 2.3 (4.3) | 49 | 2 | 93 | 11 | >99 |
| 2 | **3** | 6.8 (12.8) | 49 | 2 | 120 | 33 | >99 |
| 3 | **3** | 6.8 (12.8) | 49 | 8 | 120 | 82 | 95 |
| 4 | **4** | 6.8 (12.8) | 65 | 8 | 117 | 42 | 94 |
| 5 | **3** | 6.8 (12.8) | 49 | 15 | 121 | 83 | 97 |
| 6^{a} | **3** | 6.8 (12.8) | 49 | 8 | 112 | 27 | >99 |
| 7^{b} | **3** | 6.8 (12.8) | 49 | 8 | 120 | >99 | 97 |

| | | | | | | | |
|---|---|---|---|---|---|---|---|
| (^{a}Reaction with an excess of amine (alcohol:amine 1:1.5); ^{b}Reaction with an excess of alcohol (alcohol:amine 1.5:1)) | | | | | | | |

### EXAMPLE 5 - Scale-up reactions with 1-dodecanol and DMA:

On a schlenk line, dodecanol (3.36 mL, 15 mmol) was introduced into a 100 mL Fisher Porter (FP) bottle and three vacuum/argon cycles were done. Afterwards, DMA (40 % wt. in water, 2.0 mL, 15 mmol), 90 mg of Co₄Ni₆@Cu₁₀ and dodecane (681 µL, 3 mmol, as internal standard) were added. The FP was closed, removed from the glovebox and immediately placed in the the center of the magnetic coil and the amplitude of the magnetic field settled to the desired value for the required time. At the end of the reaction, the FP was cooled down and pressure was released. Temperature was recorded on the surface of the external glass of the FP with an IR camera after 1 h of reaction (entry 1). Catalyst was recycled (indicated as 3^{R}) and tested under the same conditions (entry 2). The results are shown in Table 6 below.

**Table 6**

| **Entry** | **Cat.** | **Cu mol % (M mol %)** | **Magnetic Field (mT)** | **Time (h)** | **Temp. (°C)** | **Conv. (%)** | **Selec. (%)** |
|---|---|---|---|---|---|---|---|
| 1 | **3** | 4.6 (8.6) | 39 | 12 | 131 | 85 | 82 |
| 2 | **3R** | 4.6 (8.6) | 39 | 12 | 134 | 89 | 76 |

## Claims

1. A process for producing alkyl amines by reacting an aliphatic alcohol with an alkyl amine in the presence of catalytic magnetic nanoparticles heated by magnetic induction.

2. The process of claim 1, wherein the catalytic magnetic nanoparticles have a magnetic core and a catalytic metal shell.

3. The process of claim 2, wherein the magnetic core is a bimetallic compound, preferably a bimetallic compound of iron and nickel, or of cobalt and nickel.

4. The process of claim 2 or 3, wherein the catalytic metal shell is a copper shell.

5. The process of any one of claims 1 to 4, wherein the magnetic nanoparticles are magnetic nanoparticles having a core of CoₓNi_{y}, wherein x is from 1 to 10, preferably equal to 4 and y = 10-x, and a shell of copper; or having a core of FeₓNi_{y}, wherein x is from 1 to 10, preferably equal to 3, and y = 10 - x, and a shell of copper, preferably the magnetic nanoparticles are Co₄Ni₆@Cu₁₀ or Fe₃Ni₇@Cu₁₀ nanoparticles.

6. The process of any one of claims 1 to 5, wherein the catalytic magnetic nanoparticles have a mean particle size of 1 to 100 nm, preferably of 5 to 50 nm, more preferably of 10 to 35 nm, determined by TEM imaging.

7. The process of any one of claims 1 to 6, wherein the aliphatic alcohol is a primary aliphatic alcohol containing 7 to 12 carbon atoms.

8. The process of any one of claims 1 to 7, wherein the alkyl amine is a dialkyl amine, preferably with alkyl groups comprising one to six carbon atoms, preferably two to four carbon atoms.

9. The process of any one of claims 1 to 8, wherein the alkyl amine is a secondary alkyl amine, and wherein preferably the two alkyl groups of the secondary alkyl amine are identical.

10. The process of any one of claims 1 to 9, wherein the aliphatic alcohol is chosen from heptanol, octanol and dodecanol, and the alkyl amine is chosen from dimethylamine, dipropylamine and dibutylamine.

11. Magnetic nanoparticles comprising a bimetallic core of CoₓNi_{y}, wherein x is from 1 to 9 and y = 10-x, and a shell of a catalytic metal, preferably of copper.

12. The magnetic nanoparticles of claim 11, answering to the formula Co₄Ni₆@Cu₁₀.

13. Magnetic nanoparticles comprising a bimetallic core of FeₓNi_{y}, wherein x is from 1 to 9 and y = 10 - x, and a shell of a catalytic metal, preferably of copper.

14. The magnetic nanoparticles of claim 13, answering to the formula Fe₃Ni₇@Cu₁₀.

15. The magnetic nanoparticles of any one of claims 11 to 14, wherein the magnetic nanoparticles have a mean particle size of 1 to 100 nm, preferably of 5 to 50 nm, more preferably of 10 to 35 nm, determined by TEM imaging.

16. The process of claim 1, wherein the catalytic magnetic particles are made of a magnetic alloy, preferably a Ni/Co alloy, more preferably Co₄Ni₆.
